# EUROPEAN PATENT APPLICATION

(11) **EP 3 957 172 A1**
(43) Date of publication of application: **23.02.2022**
(21) Application number: 20192097.2
(22) Date of filing: 21.08.2020
(51) Int. Cl.: A01M 13/00, A01M 1/20

(54) **DEVICE FOR EVAPORATING VOLATILE SUBSTANCES**

(71) Applicant: Zobele Holding SpA, 38100 Trento (IT)
(72) Inventor: DEFLORIAN, Stefano, 38100 TRENTO (IT); SORDO, Walter, 38100 TRENTO (IT)
(74) Representative: Herrero & Asociados, S.L.

(57) **Abstract**

The device for evaporating volatile substances comprises a porous mat (1) impregnated with volatile substances, a heating element (2) that heats the porous mat (1) for evaporating the volatile substances, and a reflective element (3) for reflecting the heat from the heating element (2) to said porous mat (1), wherein the reflective element (3) forms a cavity defined by a bottom and side walls where the porous mat (1) and the heating element (2) are placed.

Thanks to this feature, the reflective element reflects or redirects the heat radiation from the heating element back towards the porous mat where the volatile substances are impregnated, and the heat energy that is going away from the porous mat is reflected back to the porous mat, so that a heating element which consumes less energy can be used for the same evaporation capacity.

## Description

The present invention refers to a device for evaporating volatile substances, comprising a porous mat impregnated with volatile substances and a heating element that heats the porous mat for evaporating said volatile substances.

### Background of the invention

Electrical volatile substance diffusers have become a common consumer product in the last decades. Most common devices are connected to the electric mains and comprise a refill containing a liquid with volatile substances and a wick that transports the liquid from the inside of the refill to the external part of the wick, where the volatile particles evaporate, or a porous mat impregnated with the volatile substances. This refill or porous mat is placed in a device that with the help of an electrical heater promotes the evaporation of the volatile substance.

Standard technology for the heater construction deal about either an electrical heater embedded in a ceramic casing or a nude electrical resistor placed inside a plastic housing. The ceramic casing and the plastic housing have the function to assure electrical insulation between the resistor and the surroundings.

The existing solutions for electrical devices for evaporating volatile substances have several drawbacks.

Firstly, they have generally big dimensions and each of their surfaces dissipate heat. Therefore, only part of the heat is directed towards the wick or porous mat where it is needed. That leads to a lower electrical efficiency of the device, that makes it especially inadequate to satisfy one of the recurrent consumer requests that is portability. Portability is of special interest for insecticides substance diffuser, in order to give protection against bugs bites in outdoor conditions.

Secondly, the heat not directed towards the wick or porous mat promotes a general heating of the device that can rise substantially the temperature of the surface of the device. This is the reason why the devices use to have a considerable dimension in order to avoid the temperature at the surface exceed a limit defined by electrical certification entities. Here, again, bigger dimension of the device is a limitation for portability.

### Description of the invention

Therefore, the objective of the present invention is to provide a device for evaporating volatile substances that permit to direct substantially all the heat provided by the heating element to the porous mat, preventing an excessive rising of the temperature of the surface of the device.

With the device of the invention said drawbacks can be solved, presenting other advantages that will be described hereinafter.

The device for evaporating volatile substances according to the present invention comprises:
- a porous mat impregnated with volatile substances,
- a heating element that heats the porous mat for evaporating the volatile substances, and
a reflective element for reflecting the heat from the heating element to said porous mat,
wherein the reflective element forms a cavity defined by a bottom and side walls where the porous mat and the heating element are placed.

According to a preferred embodiment, the reflective element is formed from a metal sheet, e.g. from a mirror sheet.

Advantageously, the cavity formed by the reflective element comprises one or more seals for making the cavity airtight.

Still furtherly advantageously, the cavity airtight is having a certain level of vacuum, preferably from 0.1-0.2 bar.

According to a preferred embodiment, the heating element is a ceramic heater.

Advantageously, the porous mat is placed on the heating element, and the device also comprises a printed circuit board where the reflective element is mounted, and the printed circuit board is preferably fed by a battery.

Still advantageously, there is an air gap between the heater and the porous mat, to allow the evaporation from this side of the porous mat, that requires less energy, preferably from 0.1 to 3 mm, more preferably between 1 and 2 mm.

Preferably, there is a gap between the printed circuit board and the reflective element.

Furthermore, the device for evaporating volatile substances according to the present invention also comprises a grill that covers the porous mat, through which the volatile substances can evaporate and that also serves as a protection against accidental contact with the porous mat, which is hot during its use.

Advantageously, the porous mat can comprise a holder for facilitating its handling.

Thanks to this feature, the reflective element reflects or redirects the heat radiation from the heating element back towards the porous mat where the volatile substances are impregnated, and the heat energy that is going away from the porous mat is reflected back to the porous mat, so that a heating element which consumes less energy can be used for the same evaporation capacity.

Furthermore, as the cavity formed by the reflective element is airtight, a more efficient heating of the porous mat is achieved. This is especially of interest for portable devices that can be exposed to uncontrolled situations where dirt may be introduced accidentally inside the device. Any matter present in the cavity will affect heat transfer, due on one side to its thermal inertia, and on the other side, due to its interaction to heat transfer irradiation between heater and reflective material.

### Brief description of the drawings

For a better comprehension of what has been disclosed, some drawings are attached in which, diagrammatically and only as a non-limitative example, a practical embodiment is shown.
Fig. 1 is an exploded perspective view of the components forming the device for evaporating volatile substances according to the present invention; and
Figs. 2 and 3 are perspective views during the placement of the porous mat of the device for evaporating volatile substances according to the present invention.

### Description of a preferred embodiment

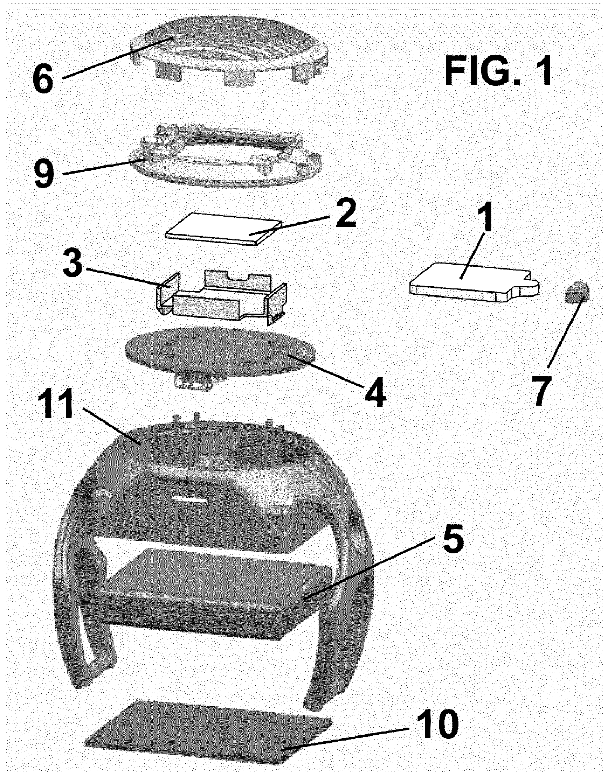

The device of Fig. 1. is an example of portable device, with the highest level of requirement for a portable device, as it is a band wrist mounted device. The requirement, as anticipated earlier are: to be lightweight, to be adequate to be use in areas where dirt or water can be present, and to have low level of external temperature of the housing.

The device for evaporating volatile substances according to the present invention comprises a porous mat 1 which is impregnated with volatile substances, such as fragrances or insecticides, and a heating element 2 that heats the porous mat 1 for evaporating the volatile substances.

The heating element 2 is any suitable heating element, but according to a preferred embodiment it is a ceramic heater, having a flat shape as shown in Fig. 1.

The porous mat 1 has also a flat shape and preferably comprises a holder 7 that facilitates its handing, because the porous mat 1 is used as a refill, so that it can be replaced once all the volatile substances have been evaporated.

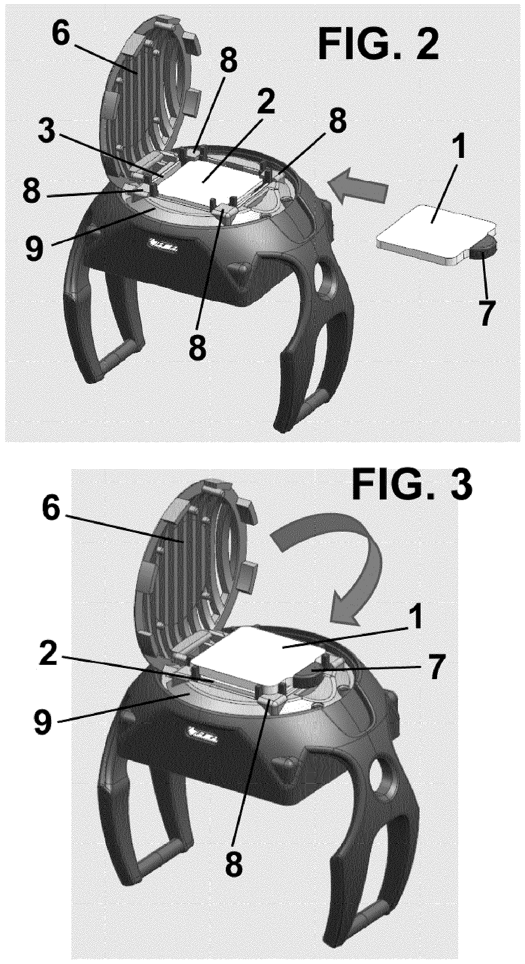

In the use position, shown in Fig. 3, the porous mat 1 is placed on the heating element 2 with a distance between them.

The device according to the present invention also comprises a reflective element 3 for reflecting the heat from the heating element 2 to the porous mat 1, and the reflective element 3 forms a cavity defined by a bottom and side walls where the porous mat 1 and the heating element 2 are placed.

According to the shown embodiment, the reflective element 3 is formed from a metal sheet, e.g. a mirror sheet, and the cavity formed by the reflective element 3 comprises one or more seals 8 for making the cavity airtight. In the shown embodiment, the seals 8 are four, each placed at the corners of the cavity, which has a rectangular plan.

The device for evaporating volatile substances according to the present invention also comprises a printed circuit board 4 where the reflective element 3 is mounted, leaving a gap between the printed circuit board 4 and the reflective element 3.

The printed circuit board 4 is fed by a battery 5, that can be connected to the mains for recharging it.

The printed circuit board 4 heats the heating element 2 placed inside the cavity defined by the reflective element 3, and this heating element 2 heats the porous mat 1 for the evaporation of the volatile substances.

The assembly of the printed circuit board 4, the reflective element 3 and the heating element 2 are mounted inside a housing 11, which can have a watch-like shape, comprising a closure 10 for accessing to the battery 5.

Furthermore, the housing 11 also comprises a grill 6 which is hinged to the rest of the housing 11 for permitting the insertion and the removal of the porous mat 1. This grill 6 also has the function of protecting the users from contacting accidentally the heated elements.

According to the shown embodiment, the grill 6 is mounted on an intermediate housing 9 that surrounds the reflective element 3.

The operation of the device according to the present invention is very simple. Firstly, a porous mat 1 must be placed in the use position, as shown in Figs. 2 and 3, and the device must be switched on.

Then the battery will feed the printed circuit board 4, which will heat the heating element 2. This heating element 2 will heat directly and indirectly the porous mat 1, and all the heat from the heating element 2 will be concentrated and reflected to the porous mat 1 by the reflective element 3.

Even though reference is made to a specific embodiment of the invention, it is clear for a person skilled in the art that the disclosed device is susceptible of variations and modifications, and that all the details cited can be substituted by other technically equivalent ones, without departing from the scope of protection defined by the attached claims.

## Claims

1. Device for evaporating volatile substances, comprising:
- a porous mat (1) impregnated with volatile substances,
- a heating element (2) that heats the porous mat (1) for evaporating the volatile substances, and
a reflective element (3) for reflecting the heat from the heating element (2) to said porous mat (1),
**characterized in that** the reflective element (3) forms a cavity defined by a bottom and side walls where the porous mat (1) and the heating element (2) are placed.

2. Device for evaporating volatile substances according to claim 1, wherein the reflective element (3) is formed from a metal sheet.

3. Device for evaporating volatile substances according to claim 1 or 2, wherein the reflective element (3) is formed from a mirror sheet.

4. Device for evaporating volatile substances according to anyone of the previous claims, wherein the cavity formed by the reflective element (3) comprises one or more seals for making the cavity airtight.

5. Device for evaporating volatile substances according to claim 4, wherein the cavity formed by the reflective element (3) has an internal vacuum in the range of -0.1 to - 0.2 bar.

6. Device for evaporating volatile substances according to claim 1, wherein the heating element (2) is a ceramic heater.

7. Device for evaporating volatile substances according to claim 1, wherein the porous mat (1) is placed on the heating element (2).

8. Device for evaporating volatile substances according to claim 1, wherein the porous mat (1) is placed at a distance of 1 to 2 mm from the heating element (2).

9. Device for evaporating volatile substances according to anyone of the previous claims, wherein the device also comprises a printed circuit board (4) where the reflective element (3) is mounted.

10. Device for evaporating volatile substances according to claim 7, wherein there is a gap between the printed circuit board (4) and the reflective element (3).

11. Device for evaporating volatile substances according to claim 7 or 8, wherein the printed circuit board (4) is fed by a battery (5).

12. Device for evaporating volatile substances according to anyone of the previous claims, also comprising a grill (6) that covers the porous mat (1).

13. Device for evaporating volatile substances according to claim 1, wherein the porous mat (1) comprises a holder (7).
